(19) Europäisches Patentamt
European Patent Office
Office européen des brevets

(11) Numéro de publication: **0 375 527 A1**

(12) # DEMANDE DE BREVET EUROPEEN

(21) Numéro de dépôt: **89403512.0**

(22) Date de dépôt: **15.12.89**

(51) Int. Cl.5: **C07D 205/08, A61K 31/395, //C07F7/18**

(30) Priorité: **19.12.88 FR 8816732**

(43) Date de publication de la demande: **27.06.90 Bulletin 90/26**

(84) Etats contractants désignés: **AT BE CH DE ES FR GB GR IT LI LU NL SE**

(71) Demandeur: **CENTRE NATIONAL DE LA RECHERCHE SCIENTIFIQUE (CNRS)**
**15, Quai Anatole France**
**F-75700 Paris Cedex 07(FR)**

(72) Inventeur: **Joyeau, Roger**
**10 rue Ponscarme**
**F-75013 Paris(FR)**
Inventeur: **Kobaiter, Randa**
**2 rue des Favorites**
**F-75015 Paris(FR)**
Inventeur: **Wakselman, Michel**
**47 rue Barrault**
**F-75013 Paris(FR)**
Inventeur: **Reboud, Michèle**
**16 rue des Orchidées**
**F-75013 Paris(FR)**

(74) Mandataire: **Mongrédien, André et al**
**c/o BREVATOME 25, rue de Ponthieu**
**F-75008 Paris(FR)**

(54) **N-Aryl-azetidinones, leur procédé de préparation et leur utilisation comme inhibiteurs des élastases.**

(57) L'invention concerne des N-aryl-azétidinones, leur procédé de préparation et leur utilisation comme inhibiteurs des élastases à sérine active.

Les N-aryl-azétidinones répondent à la formule :

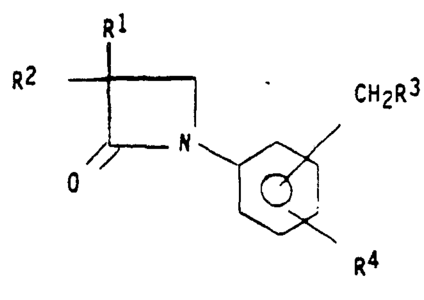

(I)

dans laquelle $R^1$ et $R^2$ peuvent être un atome d'halogène ou un radical organique, $R^3$ représente un atome d'halogène ou un autre bon groupe partant et $R^4$ représente H ou un radical organique.

Les composés avec $R^3$ = Cl ou $OSO_2CH_3$, $R^4$ = H et $R^1 = R^2$ = F ou $R^1$ = F et $R^2$ = Br sont des inhibiteurs irréversibles et sélectifs des élastases.

EP 0 375 527 A1

Xerox Copy Centre

**N-aryl-azétidinones, leur procédé de préparation et leur utilisation comme inhibiteurs des élastases.**

La présente invention concerne de nouvelles N-aryl-azétidinones, leur procédé de préparation et leur utilisation comme inhibiteurs des élastases à sérine active.

De façon plus précise, elle concerne des N-aryl-azétidinones fonctionnalisées par des substituants appropriés, leur donnant des propriétés de sélectivité vis-à-vis des élastases, en particulier de l'élastase leucocytaire et de l'élastase pancréatique, et leur conférant la capacité d'inhiber ces élastases de façon irréversible.

Le déséquilibre entre les concentrations des élastases et de leurs inhibiteurs naturels macromoléculaires est à l'origine de nombreux processus pathologiques.

Ainsi, la dégradation accrue de l'élastine du tissu conjonctif et des cartilages par l'élastase leucocytaire est impliquée dans l'emphysème pulmonaire, l'arthrite rhumatoïde, dans divers autres processus inflammatoires et dans le vieillissement cutané.

Aussi, des recherches ont été entreprises pour trouver des inhibiteurs synthétiques irréversibles de l'élastase leucocytaire qui pourraient pallier la déficience ou l'inefficacité des inhibiteurs naturels et avoir donc un grand intérêt en thérapie. Des inhibiteurs synthétiques de ce type sont décrits par W.C. Groutas dans Med. Res. Review 1987, 7, n° 2, 227-241 et par Trainor dans Trends in Pharmacological Sciences -August 1987, vol. 8, n° 8, p. 303-307.

Parmi ces inhibiteurs, on trouve des céphalosporines modifiées qui peuvent inhiber l'élas tase leucocytaire humaine, comme il est décrit par Doherty et al dans Nature, vol. 322 (1986) p. 192-194, et par Navia et al dans Nature, vol. 327 (1987), p. 79-82.

Ces céphalosporines répondent à la formule :

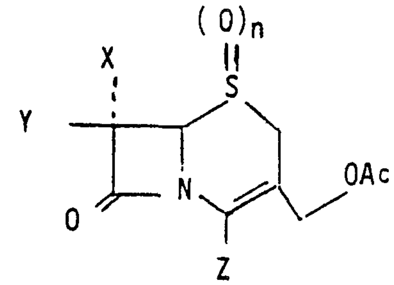

dans laquelle X peut être un atome d'hydrogène, de fluor ou de chlore ou d'autres radicaux tels que des radicaux alkoxy, aryle, $CH_3CONH$, $CF_3CONH$ ou HCONH, Y peut être un atome d'hydrogène ou d'autres radicaux organiques et Z est un radical carboxyle, ester, amide ou un atome d'hydrogène.

Zrihen et al, dans Eur. J. Med. Chem. - Chim. Ther. (1983) - 18, n° 4, p. 307-314 décrivent un mécanisme possible d'inactivation irréversible des β-lactamases par des N-aryl azétidinones fonctionnalisées. Cependant, les résultats du tableau 1 montrent que les dérivés halométhylés des N-aryl azétidinones qui possèdent une fonction électrophile latente, ne sont pas des inhibiteurs irréversibles mais des inhibiteurs compétitifs de ces enzymes.

Des études ultérieures effectuées sur certaines N-aryl-azétidinones activées ont montré que ces composés pouvaient être aussi des inhibiteurs compétitifs des β-lactamases, comme il est décrit par Joyeau et al dans Journal of Medicinal Chemistry, (1988), vol. 31, n° 2, p.370-374.

Ces dernières N-aryl-azétidinones répondent à la formule :

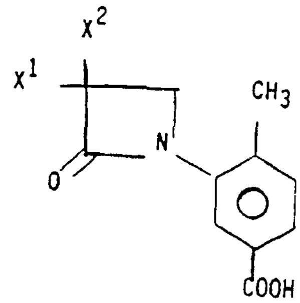

dans laquelle $X_1$ et $X_2$ peuvent représenter Br et H, Br et F, F et H ou F et F.

Ces composés présentent une affinité pour la bêta-lactamase mais ils ne sont pas des substrats de cette enzyme.

La présente invention a pour object de nouvelles N-aryl-azétidinones substituées, qui présentent la propriété d'être des inhibiteurs irréversibles des élastases à sérine active telles que l'élastase leucocytaire et l'élastase pancréatique.

Selon l'invention, les N-aryl-azétidinones répondent à la formula :

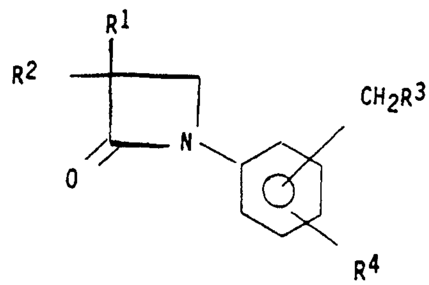

(I)

dans laquelle

-$R^1$ et $R^2$ qui peuvent être identiques ou différents, représentent un atome de F, Br, Cl ou I, ou un radical de formule $CF_3$, $COOR^5$, CN, $CONHR^5$ ou $COR^5$ avec $R^5$ représentant un radical alkyle ou aryle,

- $R^3$ représente un atome de fluor, de chlore, de brome ou d'iode, ou un radical de formule $OC(O)R^6$, $OSO_2R^6$, $OP(O)R^6{}_2$ ou $S^+R^6{}_2$ avec $R^6$ représentant un radical alkyle, perfluoroalkyle ou aryle, et

- $R^4$ représente un atome d'hydrogène ou un radical choisi parmi les radicaux alkyle et les radicaux de formule $COOR^7$, $CONHR^7$, $NO_2$, $CF_3$, CN, $SO_2R^7$, $(CH_2)_nOR^7$ et $OR^7$ avec $R^7$ représentant un atome d'hydrogène ou un radical alkyle ou aryle et n étant un nombre entier de 1 à 18.

Dans la formule des N-aryl-azétidinones de l'invention, le choix des substituants $R^1$, $R^2$ et $R^3$ permet, d'une part, de conférer à la molécule une sélectivité vis-à-vis des élastases par rapport aux autres protéases, et, d'autre part, de lui faire jouer le rôle d'inhibiteur-suicide. Le mécanisme d'action comme inhibiteur correspond probablement au démasquage de la fonction électrophile latente et substitution par un résidu nucléophile présent dans le centre actif, selon le schéma suivant :

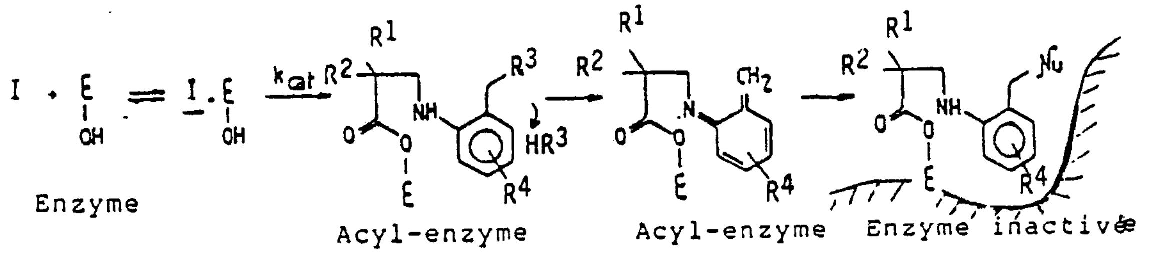


En effet, dans l'acyl-enzyme, l'halogénure amino-benzylique possédant un bon groupe partant $R^3$ est très réactif. La substitution par un résidu nucléophile Nu du centre actif de l'enzyme s'effectue rapidement par un mécanisme dissociatif (élimination-addition) impliquant comme intermédiaire une méthylène quinonimine. L'inhibiteur est retenu dans le centre actif, grâce à l'enchaînement covalent pendant toute la durée de

vie de l'acyl-enzyme. L'alkylation du résidu Nu conduit donc à une inhibition irréversible de l'élastase.

Dans ce mécanisme du type "suicide", la fonction réactive est latente et n'est libérée qu'au cours du processus catalytique de l'enzyme cible, à l'intérieur même du centre actif, ce qui limite considérablement la possibilité de réactions entre l'inhibiteur et d'autres composés présents sur son trajet in vivo.

Pour que ces N-aryl-azétidinones puissent jouer le rôle d'inhibiteur suicide, il est nécessaire que le substituant $-CH_2R^3$ soit en position ortho ou para par rapport à N. De préférence, le substituant $-CH_2R^3$ est en position ortho par rapport à N.

Bien que $R^4$ puisse représenter divers substituants, on peut obtenir de bons résultats en utilisant pour $R^4$ un atome d'hydrogène.

Les substituants $R^3$ utilisés dans l'invention sont choisis de façon à constituer de bons groupes partants favorisant le mécanisme de substitution par un résidu nucléophile. De bons résultats sont obtenus lorsque $R^3$ représente Cl ou $OSO_2CH_3$.

Lorsque les N-aryl-azétidinones de l'invention ne sont pas utilisées comme inhibiteurs d'élas tases, $R^3$ peut représenter un atome de fluor ; de telles N-aryl-azitidinones sont utilisables en particulier comme substrats des élastases.

Dans l'invention, $R^1$ et $R^2$ sont choisis pour conférer à la N-aryl-azétidinone une sélectivité vis-à-vis des élastases à inhiber. A titre d'exemples, lorsque l'élastase est l'élastase leucocytaire ou l'élastase pancréatique, $R^1$ et $R^2$ peuvent représenter tous deux un atome de fluor ou $R^1$ peut représenter un atome de fluor et $R^2$ un atome de brome.

Dans l'invention, les radicaux alkyle ou perfluoroalkyle utilisés pour $R^5$ et $R^6$ peuvent être des radicaux linéaires ou ramifiés. Ils ont généralement de 1 à 18 atomes de carbone.

Lorsque $R^5$ ou $R^6$ représente un radical aryle, celui-ci peut avoir de 6 à 14 atomes de carbone. A titre d'exemple de tels radicaux, on peut citer les radicaux phényle et naphtyle.

Lorsque dans l'invention $R^4$ ou $R^7$ représente un radical alkyle, celui-ci peut être linéaire ou ramifié. Généralement, on utilise un radical alkyle de 1 à 18 atomes de carbone.

Lorsque $R^7$ représente un radical aryle, on peut utiliser les radicaux aryle décrits précédemment.

Les N-aryl-azétidinones de l'invention répondant à la formule (I) peuvent être préparées par des procédés classiques.

A titre d'exemple, on peut préparer tout d'abord une N-aryl-azétidinone de formule :

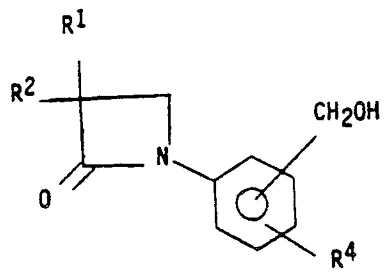

(II)

dans laquelle $R^1$, $R^2$ et $R^4$ ont la signification donnée ci-dessus, puis transformer cette N-aryl-azétidinone de formule (II) en N-aryl-azétidinone de formule (I) par réaction avec un réactif approprié choisi en fonction du groupement $R^3$ à introduire.

La N-aryl-azétidinone de formule (II) peut être préparée :

a) en faisant réagir un halogénure de $\beta$-halogénopropanoyle de formule :

$X^1OC-C(R^1R^2)-CH_2X^2$ (III)

dans laquelle $X^1$ représente F, Cl, Br ou I, $X^2$ représente Cl, Br ou I et $R^1$ et $R^2$ ont la signification donnée ci-dessus, avec une silyloxyméthylaniline de formule :

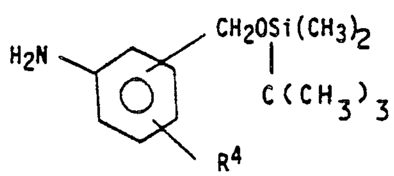

(IV)

pour obtenir un halopropionanilide de formule :

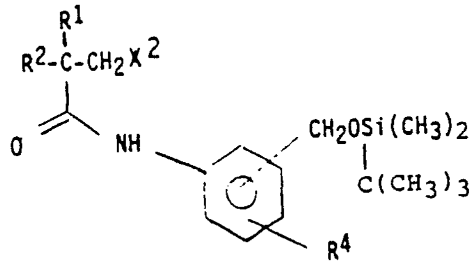

(V)

puis

b) en formant une N-aryl-azétidinone de formule :

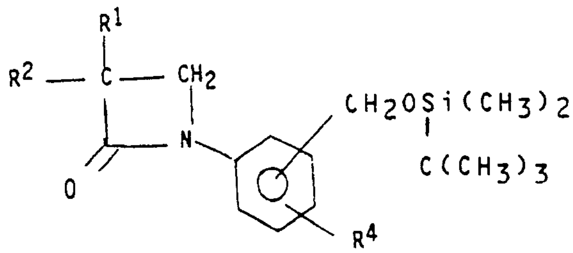

(VI)

par cyclisation de cet halopropionanilide de formule (V), et

c) en transformant cette N-aryl azétidinone de formule (VI) en N-aryl azétidinone de formule (II) par réaction avec un mélange d'acide fluorhydrique et d'eau.

Comme on l'a vu précédemment, le réactif utilisé pour transformer la N-aryl-azétidinone de formule (II) en N-aryl-azétidinone de formule (I) dépend du substituant $R^3$ à introduire.

Lorsque $R^3$ représente Cl, le réactif utilisé peut être $SOCl_2$.

Lorsque $R^3$ représente F, le réactif utilisé peut être le trifluoruré de diéthylamino soufre.

Lorsque $R^3$ représente Br ou I, le réactif utilisé peut être $PBr_3$, $SOBr_2$, $R_3SiX$(X = Br ou I et R = alkyle)-,$\Phi_3P + I_2$ + imidazole.

Lorsque $R^3$ représente $OC(O)R^6$, $OSO_2R^6$ ou $OPOR^6{}_2$, on peut utiliser comme réactif les chlorures ou anhydrides des acides correspondants. A titre d'exemple, pour introduire un substituant $R^3$ de formule $OSO_2R^6$, on peut utiliser comme réactif $ClSO_2R^6$.

Lorsque $R^3$ représente $S^+R^6_2$ la réaction utilisée peut être celle du dérivé halogéné correspondant avec un sulfure de dialkyle.

Les réactifs de départ utilisés pour la préparation des N-aryl-azétidinones sont des produits du commerce ou peuvent être préparés par des procédés classiques.

A titre d'exemple, les halogénures de bêta-bromopropanoyle de formule (III) peuvent être préparés à partir d'esters de formule $BrCH_2COCO_2C_2H_5$ par des réactions de fluoration et/ou de chloration.

Ils sont décrits dans J. Med. Chem. 1988, 31, 370 dans le cas où X est Cl et $R^1$ et $R^2$ sont F.

Dans le cas, où X est Br et $R^1$ et $R^2$ sont respectivement $B_r$ et F, on peut utiliser le procédé décrit par Molines et al dans Synthesis, 1985, p. 755.

Les silyloxyméthylanilines de formule (IV) peuvent être préparées à partir des alcools aminobenzyliques correspondants par réaction avec un halogénure de silyle en présence d'imidazole.

Les N-aryl-azétidinones de formule (I) dans laquelle $R^3$ est un atome de brome et $R^4$ représente un atome d'hydrogène ou un radical choisi parmi les radicaux de formule $COOR^7$, $CONHR^7$, $NO_2$, $CF_3$, CN et $SO_2R^7$, avec $R^7$ représentant un atome d'hydrogène ou un radical alkyle ou aryle et n étant un nombre entier de 1 à 18 peuvent aussi être préparées par un procédé consistant à faire réagir une N-aryl-azétidinone de formule :

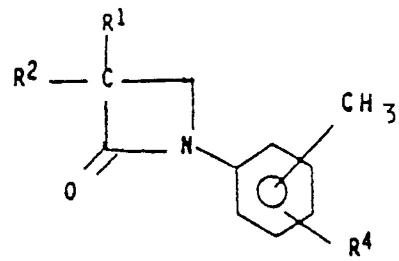

(VII)

dans laquelle $R^1$, $R^2$ et $R^4$ ont la signification donnée ci-dessus avec le N-bromosuccinimide.

La N-aryl azétidinone de formule (VII) utilisée dans ce procédé peut être préparée par un procédé analogue à celui utilisé pour la N-aryl azétidinone de formule (VI) en faisant réagir un halogénure de $\beta$-halogénopropanoyle répondant à la formule (III) donnée ci-dessus avec une toluidine de formule :

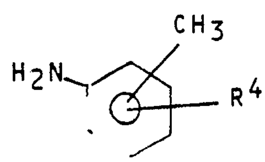

dans laquelle $R^4$ a la signification donnée ci-dessus.

Comme on l'a vu précédemment, les N-aryl-azétidinones de l'invention peuvent être utilisées dans des compositions pharmaceutiques comme inhibiteurs d'élastases.

Aussi, la présente invention a également pour objet une composition pharmaceutique comprenant un inhibiteur d'élastase, caractérisée en ce que cet inhibiteur d'élastase est une N-aryl-azétidinone de formule :

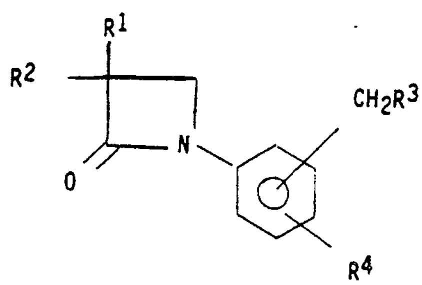

(I)

dans laquelle

- $R^1$ et $R^2$ qui peuvent être identiques ou différents représentent un atome de F, Br, Cl ou I, ou un radical de formule $CF_3$, $COOR^5$, CN, $CONHR^5$ ou $COR^5$ avec $R^5$ représentant un radical alkyle ou aryle,
- $R^3$ représente un atome de chlore, de brome ou d'iode, ou un radical de formule $OC(O)R^6$, $OSO_2R^6$, $OP(O)R^6{}_2$ ou $S^+R^6{}_2$ avec $R^6$ représentant un radical alkyle, perfluoroalkyle ou aryle, et
- $R^4$ représente un atome d'hydrogène ou un radical choisi parmi les radicaux alkyle et les radicaux de formule $COOR^7$, $CONHR^7$, $NO_2$, $CF_3$, CN, $SO_2R^7$, $(CH_2)_nOR^7$ et $OR^7$ avec $R^7$ représentant un atome d'hydrogène ou un radical alkyle ou aryle et n étant un nombre entier de 1 à 18.

Ces compositions pharmaceutiques peuvent être sous la forme de solutions, de suspensions, de poudres ou de granulés solubilisables, de sirops ou d'élixirs, de gouttes auriculaires, nasales ou ophtalmiques, de comprimés, de gélules, d'aérosols, de pommades, d'applications transdermiques ou de suppositoires dans des présentations dosées contenant des supports non toxiques, des adjuvants et des excipients. Les injections peuvent être par exemple intraveineuses, intramusculaires, sous-cutanées, intradermiques, intrasternales, intra-articulaires ; des techniques d'infusion ou des techniques d'instillation (intratrachéale par exemple) peuvent être utilisées.

En vue de cibler plus particulièrement le tissu pulmonaire, des solutions contenant des microsphères d'albumine sur lesquelles les N-aryl-azétidinones sont liées de façon covalente peuvent être réalisées.

Les préparations pour usage oral peuvent contenir un ou plusieurs agents de sucrage, agents d'aromatisation et agents de conservation. Les comprimés contiennent la molécule active de N-aryl-azétidinone mélangée à des excipients non toxiques et acceptables sur le plan pharmaceutique. Parmi les excipients, on peut citer par exemple des diluants inertes comme le carbonate de calcium ou de sodium, le phosphate de calcium ou de sodium, le lactose ; des agents permettant la granulation et l'effritement, par exemple, l'amidon de maïs ; des agents de fixation, par exemple la gélatine, l'amidon ; des agents de lubrification, par exemple le talc ou le stéarate de magnésium. Les comprimés peuvent être enrobés ou non enrobés (par exemple à l'aide de monostéarate ou de distéarate de glycérol) afin de retarder leur désintégration et leur absorption.

Les gélules peuvent présenter une capsule de gélatine dure contenant la molécule active mélangée à un solide inerte (kaolin, carbonate de calcium par exemple), ou une capsule de gélatine souple dans laquelle la N-aryl-azétidinone est mélangée à de l'eau ou à un corps gras (paraffine liquide par exemple).

Des aérosols de trois types peuvent être en particulier envisagés : (a) aérosols aqueux (administrés à l'aide de nébuliseurs) pour lesquels une meilleure solubilisation des N-aryl-azétidinones peut être obtenue par l'addition d'un cosolvant ou par formation de micelles ; (b) aérosols pressurisés avec par exemple comme gaz vecteurs des hydrocarbures chlorés et fluorés de différentes formules (ou leurs substituts) dans lesquels les N-aryl-azétidinones peuvent être dissoutes ou suspendues ; (c) aérosols en poudre avec les N-aryl-azétidinones en fines particules dans une capsule de gélati ne par exemple.

Des suspensions aqueuses contenant des N-aryl-azétidinones et des excipients convenables, avec éventuellement un ou plusieurs agents de conservation (par exemple, l'éthyl p-hydroxybenzoate), agents de coloration, agents sucrés et agents d'aromatisation peuvent être réalisées. Parmi les excipients, on peut citer des agents de suspensions (par exemple, la méthylcellulose, la gomme d'acacia), des agents de dispersion ou de mouillage tels que, par exemple, des phosphatides naturels (exemple : lécithine) ou des produits de condensation de l'oxyde d'éthylène avec divers esters partiels d'acides gras ou alcools aliphatiques. Des suspensions huileuses de la molécule active peuvent être préparées en utilisant une huile végétale (huile d'olive par exemple) ou une huile minérale (paraffine liquide par exemple), en présence éventuellement d'agents de sucrage, d'aromatisation comme ceux donnés en exemple plus haut et d'agents de conservation (en particulier, un antioxydant).

Sirops et élixirs pourront contenir des agents de sucrage (sorbitol, sucrose par exemple), un ou plusieurs agents de conservation et d'aromatisation. Des granules ou des poudres susceptibles d'être mises en suspension dans l'eau peuvent être obtenues par mélange des N-aryl-azétidinones avec un agent mouillant ou dispersif, un ou plusieurs agents de conservation et divers excipients. Des émulsions des N-aryl-azétidinones dans l'eau peuvent être réalisées en utilisant une huile minérale ou végétale et divers agents émulsifiants tels que, par exemple, des gommes naturelles, des phosphatides naturels, divers acides gras estérifiés.

Les N-aryl-azétidinones peuvent aussi être présentées sous forme de suspensions stériles injectables, aqueuses ou huileuses, utilisant les agents de suspension ou de mouillage décrits plus haut. Les solvants ou diluants ou excipients peuvent être par exemple le 1,3-butanediol, une solution isotonique de chlorure de sodium, l'eau, etc.... Les suppositoires contenant le principe actif peuvent être préparés avec les excipients habituels dans ce domaine tels que le polyéthylène glycol ou le beurre de cacao par exemple. Pour les usages locaux, des pommades, des crèmes, des gelées, des suspensions, des solutions, etc. ... contenant le principe actif peuvent être préparées.

Des doses de 0,1 à 40mg par kilogramme de poids corporel et par jour peuvent être proposées étant entendu cependant que la dose pour un patient donné peut dépendre d'un certain nombre de facteurs tels que par exemple l'efficacité de la N-aryl-azétidinone concernée, l'âge, le poids, le mode d'administration, le régime, les interactions médicamenteuses, la gravité de la maladie.

Ces compositions sont utilisables en particulier pour le traitement des processus inflammatoires aigus ou chroniques et des processus dégénératifs quel que soit l'organe concerné tels que l'emphysème pulmonaire, l'inflammation bronchique, l'arthrite rhumatoïde, l'arthrite infectieuse, la fièvre rhumatoïde, le vieillissement cutané, la périodontite, la gingivite, l'athérosclérose, les glomérulonéphrites, le syndrome de détresse respiratoire, le choc septique, la maladie de Crohn, la goutte, la pancréatite et les maladies assimilées.

D'autres caractéristiques et avantages de l'invention apparaîtront mieux à la lecture des exemples suivants.

**Exemples1 à 7.**

Ces exemples illustrent la préparation de trois N-aryl-azétidinones de l'invention suivant le schéma réactionnel suivant :

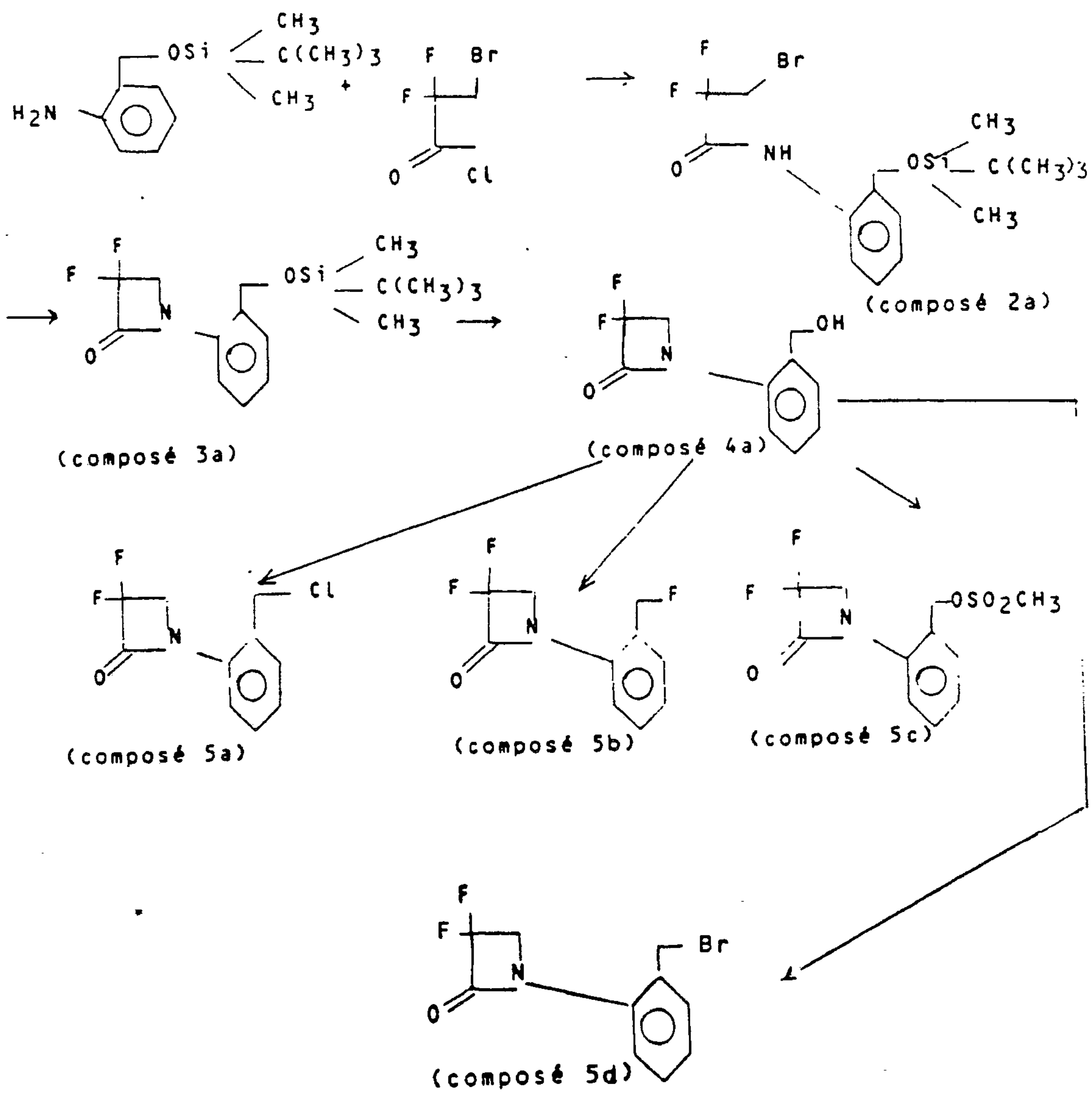

(composé 2a)

(composé 3a)

(composé 4a)

(composé 5a)

(composé 5b)

(composé 5c)

(composé 5d)

**Exemple 1** : Préparation de N-(tert-butyldiméthylsilyloxyméthyl-2 phényl)difluoro-2,2 bromo-3 propionamide (composé n° 2a)

Pour cette synthèse on utilise la tert-butyl diméthylsilyloxyméthyl-2 aniline préparée selon la méthode décrite par G. Just et R. Zamboni dans Canad. J. Chem. 1978, 5b, p. 2720, et du chlorure de bromo-3 difluoro-2,2 propanoyle préparé par le procédé décrit par Joyeau et al dans Journal of Medicinal Chemistry, 1988, vol 31, n° 2, p. 372-373.

On ajoute goutte à goutte à 4°C à une solution du chlorure de bromo-3-difluoro-2,2 propanoyle (1,1éq.)dans du toluène sec (2ml/mmol) un mélange équimolaire de triéthylamine et de la tert-butyl diméthylsilyloxyméthyl-2 aniline dissous dans du toluène (1ml/mmol). On maintient la température à 15-20°C pendant 35min. On reprend le mélange réactionnel à l'éther, on le lave avec une solution aqueuse saturée de $NaHCO_3$ puis par une solution aqueuse saturée de NaCl jusqu'à la neutralité. On sèche la phase organique sur $MgSO_4$ et on l'évapore.

On purifie le résidu obtenu par chromatographie Flash en utilisant un mélange éther/pentane avec un rapport en volume 1/6. On obtient ainsi 560mg de N-(tert-butyldiméthylsilyloxyméthyl-2 phényl)difluoro-2,2 bromo-3 propionamide, ce qui correspond à un rendement de 68%.

Les caractéristiques du produit obtenu sont les suivantes :

Point de fusion : 36,8°C.

Analyse infrarouge : $IR(CH_2Cl_2)$ : 3400 ; 1700$cm^{-1}$

Analyse par résonance magnétique nucléaire : $^1$HRMN $(CD_3)_2CO$ : 0,17ppm (6H, s) ; 0,96 (9H, s) ; 4,16 (2H, t, J = 14,07Hz) ; 4,94 (2H, s) ; 7,4 ($3H_{arm}$, m) ; 8,07 (1H, NH).
$^{19}$FRMN $\delta(CFCl_3)$ : - 105,6ppm (2F, t, J = 14,1Hz).

| Analyse élémentaire : | | |
|---|---|---|
| | trouvée | calculée |
| C | 47,28 | 47,06 |
| H | 5,94 | 5,92 |
| N | 3,37 | 3,43 |

m/z : 393-395 (M-14) ; (350-352) ; (230-228) ; 130 ; 91 ; 77.

**Exemple 2 :** Préparation de la N-(tertbutyldiméthylsilyloxyméthyl-2 phényl)difluoro-3,3 azétidin-2 one (composé 3a).

On dissout 1,5mmol du composé n° 2a obtenu dans l'exemple 1 dans 9ml d'un mélange de diméthylformamide (DMF) et de $CH_2Cl_2$ avec un rapport en volume de 1/6. On ajoute ensuite la solution sur une durée de 40min à -10° C, à une suspension de NaH (60% en dispersion dans l'huile ; 3,5eq) dans le même mélange de solvant DMF : $CH_2Cl_2$ (9ml).

Après agitation du milieu réactionnel pendant 35 à 45min, on lave rapidement le mélange réactionnel avec une solution aqueuse saturée de chlorure d'ammonium jusqu'à la neutralité. On sèche sur $MgSO_4$ et on évapore à la pompe à palettes. On purifie ensuite le résidu sur une colonne de Florisil en utilisant un mélange éther/pentane (1 :12). On obtient ainsi 303mg du composé n° 3a sous la forme d'un solide blanc, ce qui correspond à un rendement de 53%.

Les caractéristiques du produit obtenu sont les suivantes :
Point de fusion : 30,2° C.
Analyse par infrarouge : IR($CH_2Cl_2$) : 1775cm$^{-1}$.
Analyse par résonance magnétique nucléaire :
$^1$HRMN $(CD_3)_2CO$ : 0,13ppm (6H, s) 0,95 (9H, s) ; 4,56 (2H, t, J = 6,78Hz) ; 4,91 (2H, s) ; 7,46 (4H, arom, m).
$^{19}$FRMN - $\delta$ $(CFCl_3)$ : - 117ppm (2F, t, J = 6,50Hz). m/z : 312 (M-15) ; 270 ; 162 ; 148 ; 117 ; 91 ; 77.

**Exemple 3 :** Préparation de la N-(hydroxyméthyl-2 phényl)difluoro-3,3 azétidin-2 one (composé n° 4a).

On dissout 0,19mmol du composé 3a dans 1ml d'acétonitrile, et on ajoute goutte à goutte cette solution à un mélange $HF/H_2O$ 40% correspondant à 3eq. On maintient le mélange sous agitation pendant 5min à la température ambiante, puis on neutralise l'excès de HF par une solution aqueuse de $NaHCO_3$ à 5%.

On reprend ensuite le mélange dans de l'éther ; on lave la phase organique rapidement avec une solution aqueuse saturée de NaCl, puis on sèche sur $MgSO_4$ et on évapore. On obtient ainsi le composé 4a sous forme d'une huile incolore. Ses caractéristiques sont les suivantes :
Analyse infrarouge : IR($CH_2Cl_2$) : 3590-3490 ($\nu$OH) ; 1768 ($\nu_{CO}$) cm$^{-1}$.
Analyse par résonance magnétique nucléaire : $^1$HRMN $(CD_3)_2CO$ : 4,58ppm (2H, t, J = 6,84Hz) ; 4,76 (2H, m) ; 7,4 (2Harm, m) ; 7,62 (2Harm, m)
$^{19}$FRMN $\delta$ $(CFCl_3)$ : - 112,66 (2F, t, J = 6,7Hz).

**Exemple 4 :** Préparation de N-(chlorométhyl-2 phényl)difluoro-3,3 azétidin-2 one (composé n° 5a).

Le réactif utilisé dans ce cas est le réactif de Vilsmaier et on le prépare en ajoutant 200µl de chlorure de thionyle ($SOCl_2$)à 1ml de diméthylformamide sec (DMF) à une température de 0 à 4° C et en agitant pendant 5min. On obtient ainsi un réactif $SOCl_2$/DMF.

53µl de ce réactif sont ajoutés goutte à goutte à 0,11mmol du composé 4a dissous dans le miniumum de DMF sec. On agite ensuite le mélange pendant 20min à la température ambiante, puis on évapore le

chlorure de thionyle sous vide et on élimine ensuite le DMF à la pompe à palettes. On purifie le résidu obtenu sur plaque préparative de gel de silice en utilisant un mélange éther/pentane avec un rapport en volume 1/1,5. On obtient ainsi 13mg du composé 5a sous forme d'huile. Ceci correspond à un rendement de 51%.

Les caractéristiques de ce composé sont les suivantes :
IR($CH_2Cl_2$) : 1780cm$^{-1}$ ($\nu_{CO}$)
$^1$HRMN $(CD_3)_2CO$) :4,6ppm (2H, t, J = 6,88Hz) ; 4,97 (2H, s) 7,63 (4$H_{arm}$, m).
$^{19}$FRMN - $\delta$($CFCl_3$) : - 112,8 (2F, t, J = 6,7Hz).
Masse moléculaire pour $C_{10}H_8ClF_2NO$
trouvée : 231,0263
calculée : 231,02625.
m/z : 231-233 ($M^+$. isotopes Cl) ; 165-167 ; 132, 118, 92, 77.

__Exemple 5 : Préparation du N-(fluorométhyl-2 phényl)difluoro-3,3 azétidin-2 one (composé n° 5b).__

On ajoute lentement une quantité équimolaire de trifluorure de diéthylamino soufre (DAST) à une solution du composé 4a dans le chlorure de méthylène sec, à -78° C sous atmosphère d'azote. On maintient le mélange réactionnel à -40° C pendant 40 à 50 min. On suit la fin de la réaction sur couche mince de silice. Après évaporation du solvant, on purifie le résidu obtenu sur plaque préparative en utilisant un mélange éther/pentane dans un rapport 1:1,5). On obtient ainsi 11mg du compose n° 5b sous forme d'une huile incolore, ce qui correspond à un rendement de 41%.

Les caractéristiques de ce composé sont les suivantes :
IR ($CH_2Cl_2$) :(1780m$^-$ ($\nu_{CO}$).
$^1$HRMN $(CD_3)_2CO$) : 4,59 (2H, t, J = 7,05Hz) ; 5,64 (2H, d, $J_{HF}$ = 47,51 Hz) ; 7,59 (4H, m).
$^{19}$FRMN - $\delta$($CFCl_3$) : 112,6ppm (2F, t, J = 6,8Hz) ; -205(1F, t, J = 47,5Hz)
Masse moléculaire :
trouvée : 215,0562
calculée : 215,05580
m/z : 215 ($M^{+\bullet}$) ; 151 ; 109.

__Exemple 6 : Préparation de N-(méthanesulfonyloxyméthyl-2 phényl) difluoro-3,3 azétidin-2 one (composé 5c).__

On dissout 0,17mmol du composé 4a dans 0,8ml de lutidine-2,6 à 0° C, puis on ajoute 0,18mmol de chlorure de méthane sulfonyle à cette solution. Après agitation du mélange réactionnel pendant 1h30min à une température de 4 à 10° C, on reprend la solution dans 1ml d'éther, puis on la lave 2 fois avec 0,5ml d'une solution aqueuse saturée de NaCl.

On sèche la phase organique sur $MgSO_4$, puis on évapore. On purifie l'huile obtenue sur plaque préparative de silice en utilisant un mélange éther : pentane dans un rapport 1 : 1. On obtient ainsi 19mg du composé 5c, ce qui correspond à un rendement de 39%.

Les caractéristiques du composé sont les suivantes :
IR ($CH_2Cl_2$) : 1780cm$^{-1}$ ($\nu$CO) ; 1350cm$^{-1}$
$^1$HRMN ($(CD_3)_2CO$) : 3,95 (3H, s) ; 4,6 (2H, t, J = 6,31 Hz)4,95 (2H, s) ; 7,6 (4H, m).
$^{19}$FRMN ($CFCl_3$) : - 118ppm (2F, t, J = 6,4Hz).

__Exemple 7 : Préparation de la N-(bromométhyl-2 phényl) difluoro-3,3 azétidin-2-one (composé n° 5d)__

On traite 60mg (0,28mmol) du composé n° 4a par 4 équivalents (0,15ml) de bromure de triméthylsilyle, à la température ambiante pendant 35 min. On purifie ensuite le composé obtenu par chromatographie sur plaque de gel de silice en utilisant un mélange éther-pentane (1:6). On obtient ainsi 16mg du composé n° 5d, ce qui correspond à un rendement de 25%.

Les caractéristiques de ce composé sont les suivantes :
IR : ($CH_2Cl_2$) : 1775 cm$^{-1}$.
$^1$H RMN $(CD_3)_2CO$ : 7,37 ppm (4H,m) ; 4,72 (2H,s) ; 4,47 (2H, t, J = 6,7Hz).
$^{19}$F RMN (/$CFCl_3$) : -115.6 ppm (t, J = 6,6 Hz).

Masse précise : (observée calculée : 274,9760/274,97579).
m/z : 275-277 ($M^+$ isotopes Br) ; 196(M-Br) ; 132 ; 91 ; 77.

**Exemples 8 à 12.**

Ces exemples correspondent aux schémas réactionnels suivants :

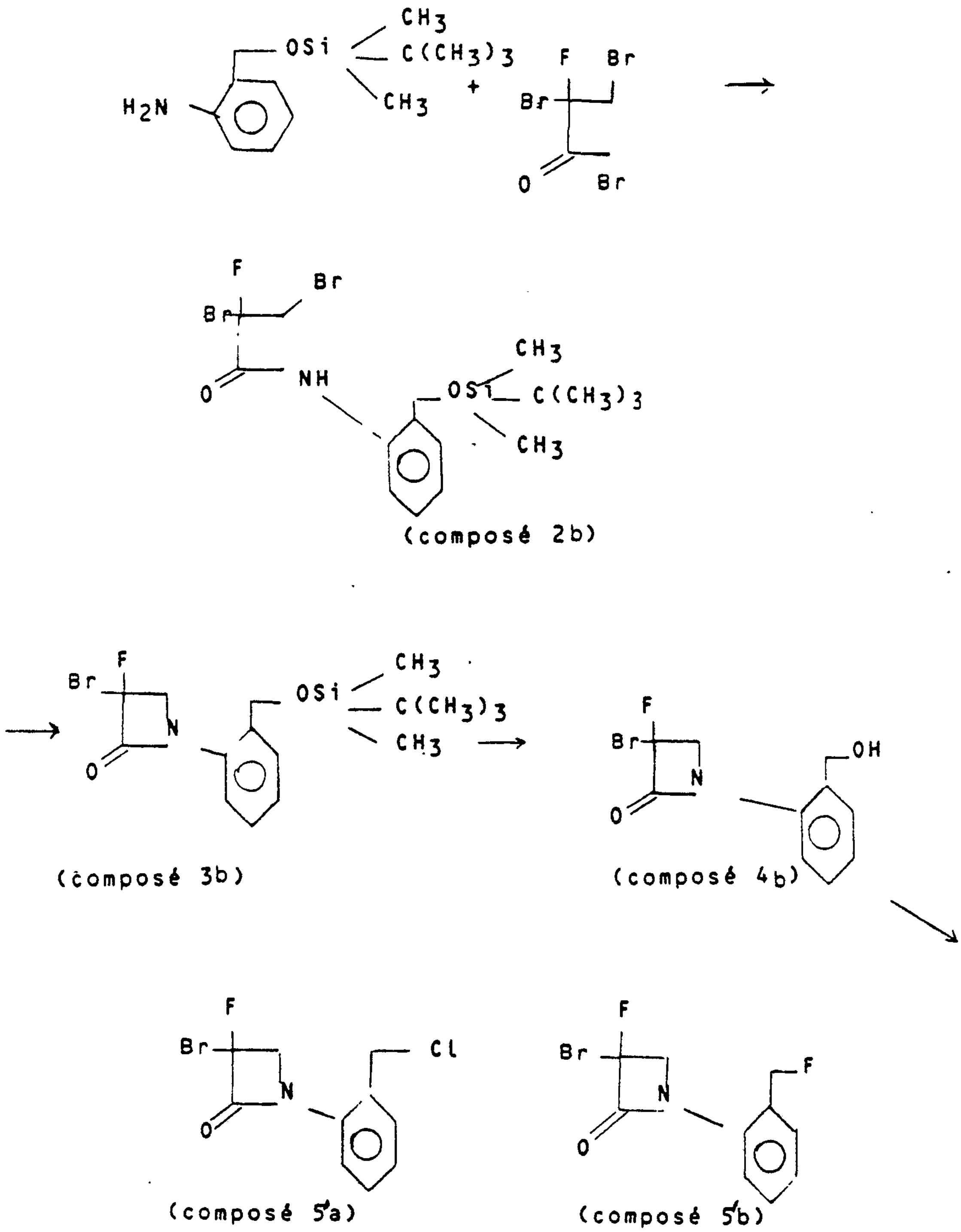

(composé 2b)

(composé 3b)

(composé 4b)

(composé 5'a)

(composé 5'b)

**Exemple 8 :** Préparation de N-(tertbutyl diméthyl silyloxyméthyl-2 phényl) dibromo-2,3 fluoro-2 propanamide (composé 2b)

On suit le même mode opératoire que dans l'exemple 1 pour préparer le composé 2b, sauf que l'on utilise 1,1eq de bromure de dibromo-2,3 fluoro-2 propanoyle au lieu de 1,1eq de chlorure de bromo-3 difluoro-2,2 propanoyle.

On obtient ainsi 882mg du composé n° 2b, ce qui correspond à un rendement de 63%.

Les caractéristiques du produit sont les suivantes :

Point de fusion : 58,2° C

IR($CH_2Cl_2$) : 3290 ; 1695 $cm^{-1}$.

$^1$HRMN $(CD_3)_2CO)$ : 0,08 (6H, s) ; 0,87 (9H, s) ; 4,40 (1H, dd, J=11,49 ; 9,01Hz) ; 4,63 (1H, dd, J=11,52 ; 31,06Hz) ; 7,3 (4H, m) ; 9,2 (1H, NH). $^{19}$FRMN $\delta$($CFCl_3$) : -118,6ppm (ddd ; 30 ; 8Hz)

| Analyse : | | |
|---|---|---|
| | observée | calculée |
| C | 41,20 | 40,95 |
| H | 5,08 | 5,16 |
| N | 2,98 | 2,98 |

m/z : 410-412-414 (M-57) ; 252 (M-2Br) 130, 91, 77.

**Exemple 9 :** Préparation du N-(tertbutyl diméthyl silyloxyméthyl-2 phényl) bromo-3 fluoro-3 azétidin-2 one (composé n° 3b).

On suit le même mode opératoire que dans l'exemple 2 pour préparer le composé 3b en utilisant 1,5mmol du composé 2b au lieu de 1,5mmol du composé 2a. Après purification sur plaque préparative de gel de silice en utilisant un mélange acétate d'éthyle pentane avec un rapport 1/10, on obtient 93mg de composé 3b sous forme d'huile. Ceci correspond à un rendement de 41%.

Les caractéristiques du produit obtenu sont les suivantes :

IR($CH_2Cl_2$) : 1770$cm^{-1}$ ;

$^1$HRMN $(CD_3)_2CO)$ : 0,14ppm (6H,s) ; 0,95 (9H, s) ; 4,6 (1H, dd ; J=7,21 (x2)Hz) ; 4,8(1H, dd, J= 7,36 ; 9,29Hz) ; 4,9 (2H ; sys (AB) ; J=13,93Hz) ; 7,35 ($2H_{am}$; m) ; 7,6 ($2H_{am}$; m).

$^{19}$FRMN $\delta$($CFCl_3$) : - 120ppm (dd ; J= 7,52 (x2) Hz)

Masse moléculaire pour $C_{16}H_{23}BrFNO_2Si$

trouvée : 387,0640

calculée : 387,0666

m/z : 388 ($M^{+\bullet}$faible) ; (373-375) (332-335) ; 132 ; 91.

**Exemple 10 :** Préparation du N-(hydroxyméthyl-2 phényl)bromo-3 fluoro-3 azétidin-2 one (composé n° 4b).

On suit le même mode opératoire que dans l'exemple 3 pour préparer le composé 4b en utilisant 0,19mmol du composé 3b au lieu de 0,19mmol du composé 3a. On obtient ainsi 32mg du composé 4b sous forme d'une huile incolore ; le rendement est de 80%.

Les caractéristiques du produit obtenu sont les suivantes.

IR($CH_2Cl_2$) : 3570 - 3460 ($\nu$OH) : 1765 ($\nu$CO)$cm^{-1}$

$^1$HRMN $(CD_3)_2CO)$ : 4,32ppm (2H, t, J=5,26Hz)4,47 (1H,dd, J=7,29 ; 7,24Hz) ; 4,64 (1H, dd, J=7,27 ; 9,34Hz)

$^{19}$FRMN-$\delta$($CFCl_3$) : - 122,33 (dd, J=7,7 ; 9,4 Hz)

m/z 273-275 ($M^{+\bullet}$(isotopes Br) : 216-194, 176, 149, 105, 118, 93, 77, 65.

**Exemple 11 :** Préparation du N-(chlorométhyl-2 phényl)fluoro-3 bromo-3 azétidin-2 one (compose 5'a).

On suit le même mode opératoire que dans l'exemple 4 pour préparer le composé 5'a en utilisant

0,11mmol du composé 4b au lieu de 0,11mmol du composé 4a. On obtient ainsi 32mg du composé 5'a sous forme d'une huile incolore ; ceci correspond à un rendement de 75%.

Les caractéristiques du produit obtenu sont les suivantes :

IR ($CH_2Cl_2$) : 1770cm$^{-1}$ ($\nu_{CO}$)

$^1$HRMN $(CD_3)_2CO$) : 4,47ppm (1H, dd, J=7,32 ; 6,93Hz) 4,73 (1H,dd, J=7,23 ; 9,37Hz) ; 4,85 (2H, dd, sys (AB) ; J=12Hz) ; 7,4 ($4H_{arm}$, m)

$^{19}$FRMN - δ($CFCl_3$) - 118,7ppm . dd, J=7 : 9,2Hz)

Masse moléculaire pour $C_{10}H_8FClBrNO$

trouvée : 290,9411

calculée : 290,94624

m/z : 290 293 295 ($M^{+\bullet}$ isotope Cl, Br) ; 216,167 ; 132,148 .

**Exemple 12 :** Préparation du N-(fluorométhyl-2 phényl) bromo-3 fluoro-3 azétidin-2 one (composé 5'b).

On suit le même mode opératoire que dans l'exemple 5 pour préparer le composé 5'b en utilisant le composé 4b au lieu du composé 4a. On obtient ainsi 12mg du composé 5'b sous forme d'huile incolore, ce qui correspond à un rendement de 64%.

Les caractéristiques du produit obtenu sont les suivantes :

IR ($CH_2Cl_2$) : 1770cm$^{-1}$ ($\nu_{CO}$)

$^1$HRMN $(CD_3)_2CO$) : 4,55ppm (1H, dd ; $J_{AB}$=7,3 ; 7,17Hz) 4,76 (1H, dd, JAB 7,3 ; 9,06Hz) ; 5,5 (2H, d, J=47,55Hz).

$^{19}$FRMN δ($CFCl_3$) : - 204,4 (1F, t, J=47,5Hz) ; - 118,4 (1F, t, J= 7,1 ; 9,23Hz).

Masse moléculaire :

trouvée : 274,9763

calculée : 274,97579

m/z : (275-277) ($M^{+\bullet}$) ; 151 , 123 ; 109 ; 96.

**Exemples 13 à 18.**

Ces exemples concernent la préparation de N-aryl-azétidinones conformes à l'invention selon le schéma réactionnel suivant :

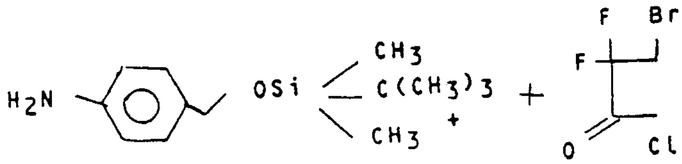

(composé 6)

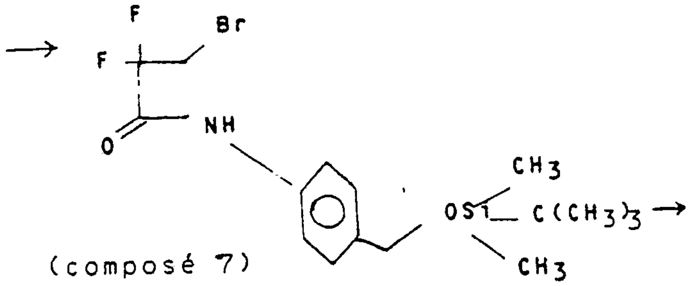

(composé 7)

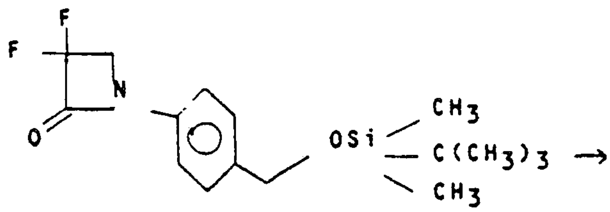

(composé 8)

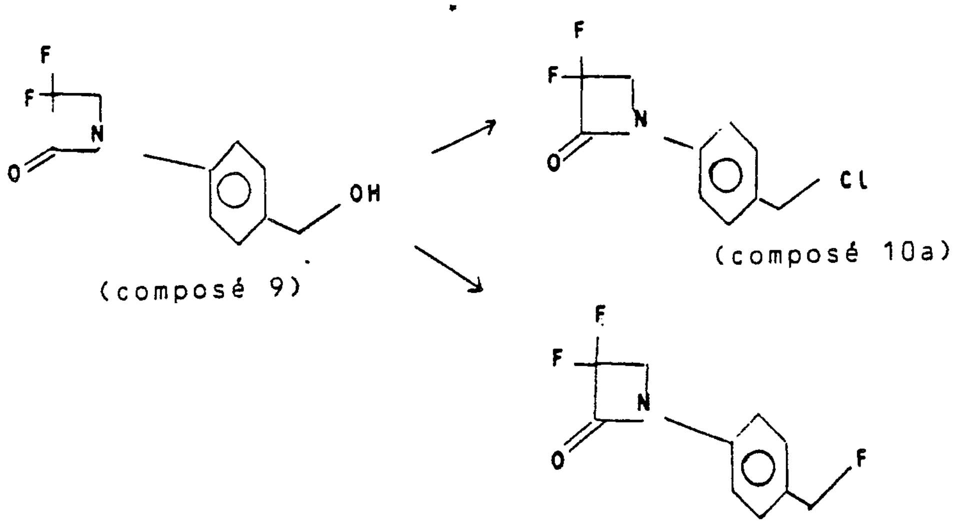

(composé 9)

(composé 10a)

(composé 10b)

**Exemple 13 :** Préparation de tert-butyl diméthyl silyloxyméthyl-4 aniline (composé n° 6).

On dissout 123mg (1mmol) d'alcool amino-4 benzylique dans 2ml de DMF sec. On ajoute à la solution 180mg (1,2mmol) de chlorure de tert-butyl diméthylsilyle puis 170mg (2,5mmol) d'imidazole.

On agite pendant 40min à la température ambiante, puis on évapore le DMF à la pompe à palettes. Le résidu est repris par l'éther (10ml) et la phase éthérée est lavée par 4x4ml eau, puis on sèche sur $MgSO_4$ et on évapore. On purifie l'huile obtenue par chromatographie Flash en utilisant un mélange éther/pentane

dans un rapport en volume de 3/2.

On obtient ainsi 173mg du composé n° 6, ce qui correspond à un rendement de 73%.

Les caractéristiques du produit obtenu sont les suivantes :

IR ($CH_2Cl_2$) : 3430-3380 ; 2800 ; 1610 ; 1510 ; 1100$cm^{-1}$

$^1$HRMN $(CD_3)_2CO$) : 0,05ppm (6H, s) ; 0,85 (9H, s) ; 4,4 (2H, $NH_2$ s large) ; 4,55 (2H, s) , 6,35 (2H, d, J=8,8Hz) ; 6,95 (2H, d, J=8,7Hz).

**Exemple 14 :** Préparation de N-(tertbutyldiméthylsilyloxyméthyl-4 phényl)difluoro-2,2 bromo-3 propionamide (composé n° 7).

On suit le même mode opératoire que dans l'exemple 1 en utilisant le composé n° 6 au lieu de la tert-butyldiméthylsilyloxyméthyl-2 aniline. On obtient ainsi 230mg du composé n° 7, ce qui correspond à un rendement de 70%.

Les caractéristiques du produit obtenu sont les suivantes :

Point de fusion : 57,3° C

IR($CH_2Cl_2$) : 3400 ; 1700$cm^{-1}$

$^1$HRMN $(CD_3)CO$) : 0,17ppm (6H, s) , 1 (9H, s) ; 4,16 (2H, t, J=14,3Hz) ; 4,82(2H, s) ; 7,43 ($2H_{am}$; d ; J=8,58Hz) ; 7,8(2H, d, J=8,53Hz).

$^{19}$FRMN - $\delta(CFCl_3)$ : - 120,8ppm (t; J=14Hz)

Masse moléculaire pour $C_{16}H_{24}F_2BrNO_2Si$ :

trouvée : 407,0729

calculée : 407,07283

| Analyse élémentaire : | | |
|---|---|---|
| | trouvée | calculée |
| C | 47,76 | 47,06 |
| H | 5,99 | 5,92 |
| N | 3,33 | 3,43 |

m/z : 407 ($M^{+\bullet}$) faible ; (352-350) isotopes Br ; (230-228) ; 106, 90.

**Exemple 15 :** Préparation de N-(tertbutyldiméthylsilyloxyméthyl-4 phényl)difluoro-3,3 azétidin-2 one (composé n° 8)

On suit le même mode opératoire que dans l'exemple 2 pour préparer le composé n° 8 en utilisant 1,5mmol de composé n° 7 au lieu de 1,5mmol de composé n° 2a. Après purification sur colonne de Florisil en utilisant un mélange éther/pentane avec un rapport en volume 1/6, on obtient 72mg du composé n° 8, ce qui correspond à un rendement de 51%. Ce composé est un solide blanc fondant à 48° C.

Les caractéristiques du produit obtenu sont les suivantes :

IR ($CH_2Cl_2$) : 1770$cm^{-1}$

$^1$HRMN $(CD_3)_2CO$) : 0,2ppm (6H, s) ; 1(9H, s) ; 4,49 (2H, t, J=6,42Hz) ; 4,87 (2H, s) ; 7,55 (4H, m).

$^{19}$FRMN $\delta(CFCl_3)$ : - 111,6ppm (2F, t, J=6,4Hz).

Masse moléculaire :

trouvée : 327,1471

calculée : 327, 14661

m/z : 327 ($M^{+\bullet}$) ; 312 ; 270 ; 196 ; 168.

**Exemple 16 :** Préparation de N-(hydroxyméthyl-4 phényl)difluoro-3,3 azétidin-2 one (composé n° 9)

On suit le même mode opératoire que dans l'exemple 3 pour préparer le composé n° 9 à partir du composé n° 8 en utilisant 0,19mmol du composé n° 8 au lieu de 0,19mmol du composé 3a. On obtient ainsi 42mg du composé n° 9 sous forme solide, ce qui correspondant à un rendement de 82%.

Les caractéristiques du produit obtenu sont les suivantes :
Point de fusion : 124 °C
IR ($CH_2Cl_2$) : 3580-3490 ($\nu$OH) : 1768 ($\nu$CO) $cm^1$
$^1$HRMN $(CD_3)_2CO$) : 4,68ppm (2H, d, J = 5,7Hz) ; 4,45 (2H, t, J = 6,32Hz) ; 4,35 (1H, t, J = 5,76Hz) ; 7,5 ($4H_{am}$ ; s)
$^{19}$FRMN - $\delta$($CFCl_3$) : - 111,6ppm (2F, t, J = 6,4Hz)
Masse moléculaire pour $C_{10}H_9F_2NO_2$
trouvée : 213,0604
calculée : 213,06014
m/z : 213($M^{+\bullet}$) ; 149 ; 106.

**Exemple 17 :** Préoaration de N-(chlorométhyl-4 phényl)difluoro-3,3 azétidin-2 one (composé n° 10a)

On suit le même mode opératoire que dans l'exemple 4 pour préparer le composé 10a à partir du composé n° 9 en utilisant 0,11mmol du composé n° 9 au lieu de 0,11mmol du composé 4a. On obtient ainsi 16mg du composé 10a sous forme de solide blanc, ce qui correspond à un rendement de 55%.

Les caractéristiques du composé obtenu sont les suivantes :
Point de fusion : 93 °C
IR ($CH_2Cl_2$) : 1770$cm^{-1}$ ( CO)
$^1$HRMN $(CD_3)_2CO$) : 4,48 (2H, t, J = 6,53Hz) ; 4,79(2H, s) ; 7,57 ($4H_{arm}$, d ; J = 8,6Hz).
$^{19}$FRMN - ($CFCl_3$) :- 111,5ppm (2F, t, J = 6,6Hz)
Masse moléculaire pour $C_{10}H_8F_2ClNO$
trouvée : 231,0260
calculée : 231,02625
m/z : 231-233 ($M^{+\bullet}$ isotope Cl) : 196 ; 167 ; 169 ; 168 ; 132 ; 118 ; 90 ; 77.

**Exemple 18 :** Préparation de N-(fluorométhyl-4 phényl)difluoro-3,3 azétidin-2 one (composé 10b).

On suit le même mode opératoire que dans l'exemple 5 pour préparer le composé 10b à partir du composé n° 9 en utilisant le composé n° 9 au lieu du composé 4a. On obtient ainsi 16mg du composé 10b sous forme solide, ce qui correspond à un rendement de 38%.

Les caractéristiques du produit obtenu sont les suivantes :
Point de fusion : 104 °C
IR ($CH_2Cl_2$) : 1770$cm^{-1}$ ( CO)
$^1$HRMN - $(CD_3)CO$) : 4,49ppm (2H, t, J = 6,49Hz) ; 5,46 (2H, d, J = 48Hz) ; 7,58 (4H, s)
$^{19}$FRMN ($CFCl_3$) : - 111,5ppm (2F, t, J = 6,6Hz) ;- 200,39 (1F, t, J = 48Hz)
Masse moléculaire
trouvée : 215,0559
calculée : 215,05580
m/z : 215 ($M^{+\bullet}$) ; 151 ; 106 ; 109

**Exemples 19 à 22.**

Ces exemples illustrent la préparation de N-aryl-azétidinones de l'invention suivant le schéma réactionnel suivant :

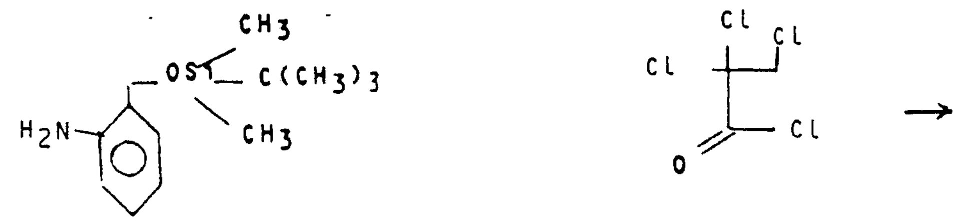

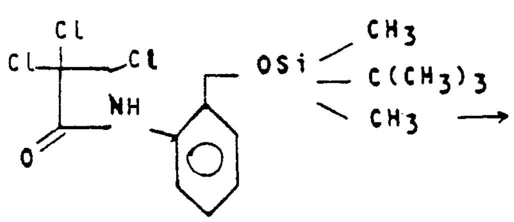

(composé 2e)

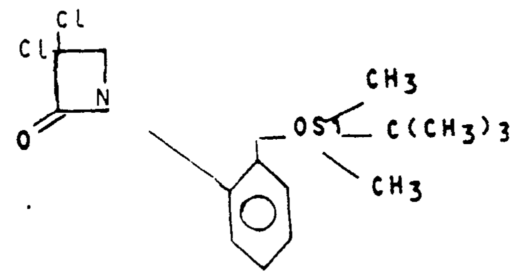

(composé 3e)

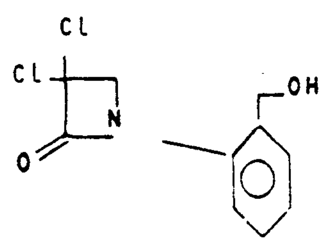

(composé 4e)

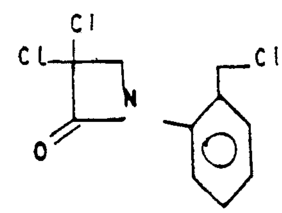

(composé 5e)

**Exemple 19 :** Préparation de N-(tert butyl diméthyl silyloxyméthyl-2 phényl) trichloro-2,2,3 propionamide (composé n° 2e).

Pour cette synthèse, on utilise la tert-butyl diméthylsilyloxyméthyl-2 aniline préparée selon la méthode décrite par G. Just et R. Zamboni dans Canad. J. Chem. 1978, 5b, p. 2720, et du chlorure de trichloropropanoyle préparé de la façon suivante.

On prépare tout d'abord le trichloro-2,2,3 propionitrile par addition de chlore sur l'α-chloroacrylonitrile comme il est décrit par H. Brintzinger et col. dans Angew. Chemie, 1948, 60, 311 ; puis on soumet le trichloro-2,2,3 propionitrile à une hydrolyse acide par $H_2SO_4$ à 60%, à 125° C pendant 5 heures pour obtenir l'acide correspondant comme il est décrit par H. Laato dans Suomen Kemistilehti, 1968, 41B, 266. On traite ensuite l'acide par $SOCl_2$ en présence d'une quantité catalytique de DMF à 40-50° C pendant une heure,ce qui donne le chlorure de trichloro-2,2,3 propanoyle qui est distillé à 50° C sous 2700 Pa (20mm de Hg). Le rendement est de 66%.

Les caractéristiques de ce composé sont les suivantes :

IR ($CH_2Cl_2$) : 1800, 1770cm$^{-1}$ (bande intense)

$^1$H RMN ($CDCl_3$) : 4,4ppm (2H,s).

On suit le même mode opératoire que dans l'exemple 1 pour préparer le N-(tert butyl diméthylsilyloxyméthyl-2 phényl) trichloro-2,2,3 propionamide à partir de la tert-butyl diméthylsilyloxyméthyl-2 aniline et du chlorure de trichloro-2,2,3 propanoyle. On obtient ainsi 814mg du composé n° 2e, ce qui correspond à un rendement de 70%.

Les caractéristiques de ce composé sont les suivantes :
Solide blanc F° = 41 °C ;
IR($CH_2Cl_2$) ; 3300 ; 1690 ; 1585 $cm^{-1}$.
$^1$H RMN : 10,25(1H,NH) ; 8,25(1H,d) ; 7,35(2H,m) ; 4,8(2H,s) ; 4,2(2H,s) ; 0,95(9H,s) ; 0,15(6H,s).
m/z : 382-384 (M-14) ; 338-340 ; 268 ; 200 ; 192 ; 164 ; 132 ; 93 ; 75 ; 29.

| Microanalyse : | | | |
|---|---|---|---|
| | observée | / | calculée % |
| C | 48,32 | / | 48,43 |
| H | 6,32 | / | 6,10 |
| N | 3,6 | / | 3,53 |

**Exemple 20 : prépar=tion de la N-(tertbutyldiméthylsilylox'méthyl-2 phényl) dichloro-3,3 azétidin-2-one (composé n° 3e).**

On suit le même mode opératoire que dans l'exemple 2 pour préparer le composé n° 3e à partir du composé n° 2e. On purifie le produit obtenu par chromatographie flash en utilisant un mélange éther / pentane (1:10). On obtient ainsi le composé n° 3e sous la forme d'un solide blanc avec un rendement de 59%.

Les caractéristiques de ce composé sont les suivantes :
IR($CH_2Cl_2$) : 1770$cm^{-1}$ ($\nu$co $\beta$lactame).
$^1$H RMN ($CDCl_3$) ; 7,5(4H, m) ; 4,8(2H,s) ; 4,5(2H,s) ; 0,95(9H,s) ; 0,15(6H,s).
Masse précise : (observée / calculée : 359,087 / 359,0875).
m/z : 344-346 ; 302-304 ; 206 ; 192 ; 132 ; 93 ; 73 ; 29 ;

| Microanalyse : | | | |
|---|---|---|---|
| | observée | / | calculée % |
| C | 53,24 | / | 53,33 |
| H | 6,62 | / | 6,43 |
| N | 3,67 | / | 3,89. |

**Exemple 21 : Préparation de la N-(hydroxyméthyl-2 phényl) dichloro-3,3 azétidin-2-one (composé n° 4e).**

On suit le même mode opératoire que dans l'exemple 3 pour préparer ce composé n° 4e à partir du composé n° 3e. On obtient ainsi le composé n° 4e sous la forme d'une huile incolore, avec un rendement de 70%.

Les caractéristiques de ce composé sont les suivantes :
IR ($CH_2Cl_2$) : 3580-3480 ($\nu$OH $cm^{-1}$ ; 1765 ($\nu$co lactame) $cm^{-1}$
$^1$H RMN ($CDCl_3$) 7,45ppm (4H,m) ; 4,75(2H,s) ; 4,5(2H,s) ; 3(1H ,s).

**Exemple 22 : Préparation de la N-(chlorométhyl-2 phényl) dichloro-3,3 azétidin-2-one (compose n° 5e).**

On suit le même mode opératoire que dans l'exemple 4 pour préparer ce composé n° 5e à partir du composé n° 4e. On purifie le produit obtenu par chromatographie sur gel de silice en utilisant un mélange éther-pentane (1:5). On obtient ainsi le composé n° 5e sous la forme d'un solide blanc avec un rendement de 67%.

Les caractéristiques de ce composé sont les suivantes :
F : 79° C
IR : ($CH_2Cl_2$) : 1770cm$^{-1}$
$^1$H RMN ($CDCl_3$) : 7,5(4H,s) ; 4,8(2H,s) ; 4,5(2H,s) ;
Masse précise : (observée : calculée 262,9675 / 262,96715).
m/z : 263-261 (isotopes Cl) ; 169-167 ; 132 ; 91 ; 77 ; 40 ; 29.

| Microanalyse : | | | |
|---|---|---|---|
| | observée | / | calculée % |
| C : | 45,67 | / | 45,4 |
| H : | 3,12 | / | 3,05 |
| N : | 5,06 | / | 5,3. |

**Exemple 23 :** Préparation de la N-(bromométhyl-2 phényl) difluoro-3,3 azétidin-2-one (composé n° 5d).

Dans cet exemple, on utilise un autre procédé pour préparer le composé n° 5d de l'exemple 7.
Ce procédé correspond au schéma réactionnel suivant :

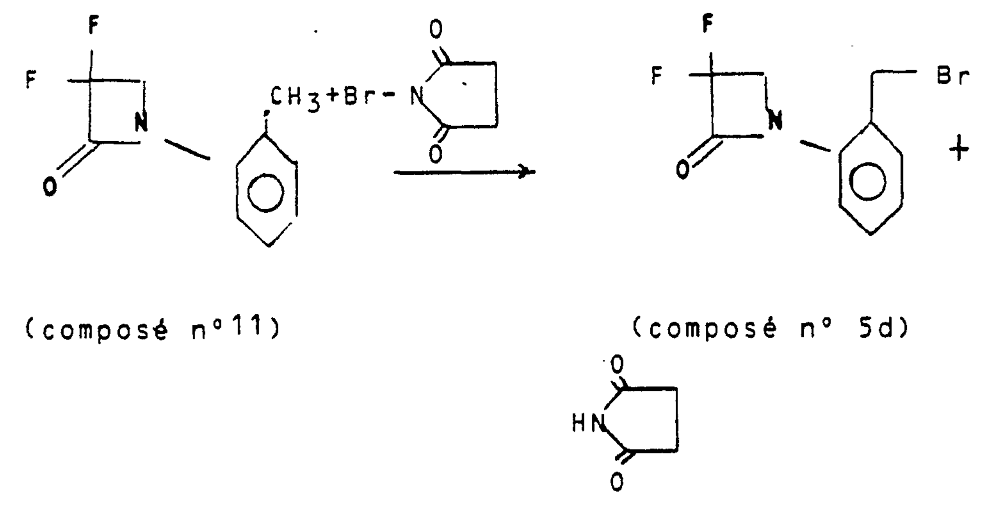

(composé n°11) (composé n° 5d)

Le composé n° 11 de départ est préparé à partir du chlorure de bromo-3 difluoro-2,2 propanoyle utilisé dans l'exemple 1 et d'ortho-toluidine par cyclisation en suivant le même mode opératoire que dans les exemples 1 et 2 pour la préparation du composé n° 3a.

On dissout 24mg (0,12mmol) du composé n° 11 dans 20ml de $CCl_4$ et on ajoute 21mg (0,12mmol) de N-bromosuccinimide et une quantité catalytique de peroxyde de benzoyle. Après avoir mis à reflux (78° C) pendant 1 heure en éclairant avec une lampe de 150 watts, on filtre à chaud le succinimide formé ; on refroidit alors le filtrat et on l'évapore sous vide, ce qui donne un produit solide que l'on purifie par chromatographie sur gel de silice. On obtient ainsi 20mg du composé n° 5d, ce qui correspond à un rendement de 62%.

**Exemple 24.**

On vérifie les propriétés des N-aryl-azétidinones obtenues dans les exemples 4, 5, 6, 7, 11, 17, 22 et 23, en particulier leur capacité d'inactiver l'élastase pancréatique porcine (PPE) et l'élastase leucocytaire humaine (HLE) en utilisant les méthodes de Kitz et Wilson pour la HLE dans le cas des composés 5a, 5c, 5d, 5e, 5a' et 10a, la méthode de Kitz et Wilson pour la PPE dans le cas des composés 5a, 5c, 5e et 10a, et la méthode de Hart et O'Brian pour la PPE dans le cas des composés 5a et 5'a. Toutes les mesures cinétiques sont effectuées à 37° C et à un pH de 8,0 avec un tampon Tris 0,1M, (PPE) ou un tampon Tris 0,1M, Brij 0,01%, $NaN_3$ 0,02% (HLE) (pH 7,5 pour le composé 5d).

La méthode de Hart et O'Brian a été publiée en 1973 dans Biochemistry, 12, 2940-2945, celle de Kitz et Wilson en 1962 dans J. Biol. Chem., 237, 3245-3249.

On détermine ainsi la constante ki de premier ordre pour la formation de l'enzyme inactivée et la constante Ki, constante de dissociation du complexe enzyme-inhibiteur. Leur rapport ki/Ki est la constante apparente de second ordre caractérisant l'inactivation.

Les résultats obtenus sont donnés dans le tableau qui suit.

Au vu de ces résultats, on constate que les composés 5a, 5c, 5'a, 5e et 10a inactivent la PPE et la HLE.

Lorsqu'on teste les composés 5a, 5c, 5'a et 10a dans des conditions analogues, comme inhibiteurs de la chymotrypsine ou de la trypsine, on constate qu'ils n'ont aucune action sur ces enzymes.

**Exemple 25.**

Dans cet exemple on teste les propriétés des composés 5b et 10b vis-à-vis de l'élastase, et l'on constate que ces composés sont des substrats des élastases mais qu'ils ne sont pas des inhibiteurs.

Ainsi, la nature du substituant $R^3$ a une influence importante sur les propriétés du produit obtenu.

**Exemple 26.**

On teste l'effet du composé 5a sur la dégradation de l'élastine, substrat naturel de l'élastase dans l'organisme humain. L'élastase préalablement traitée par le composé 5a perd sa capacité à dégrader l'élastine.

D'autre part, les composés 5a, 5c et 5'a demeurent capables d'inactiver l'élastase préalablement incubée pendant 30min en présence d'un excès d'élastine (2mg).

La toxicité aiguë du composé 5a recherchée sur des souris mâles et femelles est supérieure à 200mg/kg.

TABLEAU

| Composé | $R^1$ | $R^2$ | $R^3$ | PPE | | | HLE | | |
|---|---|---|---|---|---|---|---|---|---|
| | | | | ki | Ki | ki/Ki | k i | Ki | ki/Ki |
| | | | | ($s^{-1}$) | (M) | ($M^{-1}s^{-1}$) | ($s^{-1}$) | (M) | ($M^{-1}s^{-1}$) |
| ORTHO | | | | | | | | | |
| 5a | F | F | Cl | 0,031 | $3.10^{-5}$ | 1033 | $5.10^{-3}$ | $1,66.10^{-5}$ | 301 |
| 5c | F | F | $OSO_2CH_3$ | 0,017 | $4,8.10^{-5}$ | 354 | 0,05 | $5.10^{-4}$ | 100 |
| 5'a | F | Br | Cl | 0,048 | $0,85.10^{-5}$ | 5647 | 0,37 | $1,17 \times 10^{-3}$ | 316 |
| 5d | F | F | Br | | | | | | 40 |
| 5e | Cl | Cl | Cl | 0,028 | $5,9.10^{-4}$ | 47 | 0,03 | $5.10^{-4}$ | 60 |
| PARA | | | | | | | | | |
| 10a | F | F | Cl | 0,075 | $6,6.10^{-4}$ | 113 | | | ~1,2 |

## Revendications

1. N-aryl-azétidinone répondant à la formule :

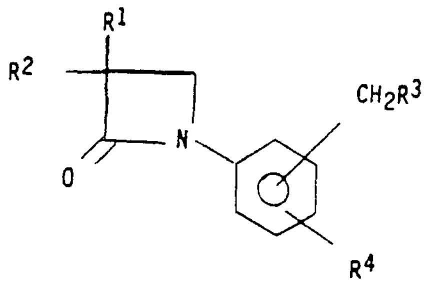

(I)

dans laquelle
- $R^1$ et $R^2$ qui peuvent être identiques ou différents, représentent un atome de F, Br, Cl ou I, ou un radical de formule $CF_3$, $COOR^5$, CN, $CONHR^5$ ou $COR^5$ avec $R^5$ représentant un radical alkyle ou aryle,
- $R^3$ représente un atome de fluor, de chlore, de brome ou d'iode, ou un radical de formule $OC(O)R^6$, $OSO_2R^6$, $OP(O)R^6{}_2$ ou $S^+R^6{}_2$ avec $R^6$ représentant un radical alkyle, perfluoroalkyle ou aryle, et
- $R^4$ représente un atome d'hydrogène ou un radical choisi parmi les radicaux alkyle et les radicaux de formule $COOR^7$, $CONHR^7$, $NO_2$, $CF_3$, CN, $SO_2R^7$, $(CH_2)_nOR^7$ et $OR^7$ avec $R^7$ représentant un atome d'hydrogène ou un radical alkyle ou aryle et n étant un nombre entier de 1 à 18.

2. N-aryl-azétidinone selon la revendication 1, caractérisée en ce que $-CH_2R^3$ est en position <u>ortho</u> ou <u>para</u> par rapport à N.

3. N-aryl-azétidinone selon la revendication 2, caractérisée en ce que $-CH_2R^3$ est en position ortho par rapport à N.

4. N-aryl-azétidinone selon l'une quelconque des revendications 1 à 3, caractérisée en ce que $R^4$ est un atome d'hydrogène.

5. N-aryl-azétidinone selon l'une quelconque des revendications 1 à 4, caractérisée en ce que $R^3$ représente F, Cl, Br ou $OSO_2CH_3$.

6. N-aryl azétidinone selon la revendication 5, caractérisée en ce que $R^1$ et $R^2$ représentent F.

7. N-aryl azétidinone selon la revendication 5, caractérisée en ce que $R^1$ représente F et $R^2$ représente Br.

8. N-aryl-azétidinone selon la revendication 5, caractérisée en ce que $R^1$ et $R^2$ représentent Cl.

9. Procédé de préparation d'une N-aryl-azétidinone répondant à la formule :

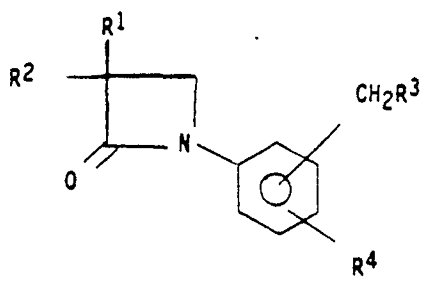

(I)

dans laquelle
- $R^1$ et $R^2$ qui peuvent être identiques ou différents représentent un atome de F, Br, Cl ou I, ou un radical de formule $CF_3$, $COOR^5$, CN ou $CONHR^5$ avec $R^5$ représentant un radical alkyle ou aryle,
- $R^3$ représente un atome de fluor, de chlore, de brome ou d'iode, ou un radical de formule $OC(O)R^6$, $OSO_2R^6$, $OP(O)R^6{}_2$ ou $S^+R^6{}_2$ avec $R^6$ représentant un radical alkyle, perfluoroalkyle ou aryle, et
- $R^4$ représente un atome d'hydrogène ou un radical choisi parmi les radicaux alkyle et les radicaux de formule $COOR^7$, $CONHR^7$, $NO_2$, $CF_3$, CN, $SO_2R^7$, $(CH_2)_nOR^7$ et $OR^7$ avec $R^7$ représentant un atome d'hydrogène ou un radical alkyle ou aryle et n étant un nombre entier de 1 à 18,

caractérisé en ce qu'il comprend les étapes successives suivantes :

1°) préparer une N-aryl-azétidinone de formule :

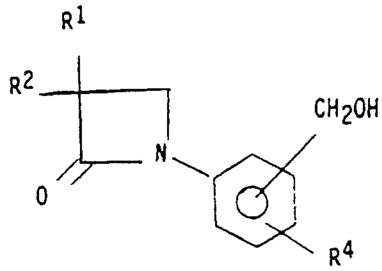

(II)

dans laquelle $R^1$, $R^2$ et $R^4$ ont la signification donnée ci-dessus, et

2°) transformer la N-aryl-azétidinone de formule (II) ainsi obtenue en N-aryl-azétidinone de formule (I) par réaction avec un réactif choisi en fonction de $R^3$.

10. Procédé selon la revendication 9, caractérisé en ce que $R^3$ représente Cl et en ce que le réactif est $SOCl_2$.

11. Procédé selon la revendication 9, caractérisé en ce que $R^3$ représente F et en ce que le réactif est le trifluorure de diéthylamino soufre.

12. Procédé selon la revendication 9, caractérisé en ce que $R^3$ représente Br et en ce que le réactif est $(CH_3)_3SiBr$.

13. Procédé selon la revendication 9, caractérisé en ce que $R^3$ représente $OSO_2R^6$ et en ce que le réactif est $ClSO_2R^6$.

14. Procédé selon l'une quelconque des revendications 9 à 13, caractérisé en ce que l'on prépare la N-aryl-azétidinone de formule (II):

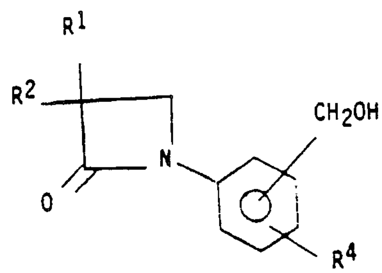

(II)

a) en faisant réagir un halogénure de β-halogénopropanoyle de formule :

$X^1OC\text{-}C(R^1R^2)\text{-}CH_2X^2$ (III)

dans laquelle $X^1$ représente F, Cl, Br ou I, $X^2$ représente Cl, Br ou I et $R^1$ et $R^2$ ont la signification donnée ci-dessus, avec une silyloxyméthylaniline de formule :

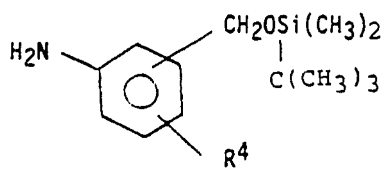

(IV)

pour obtenir un halopropionanilide de formule :

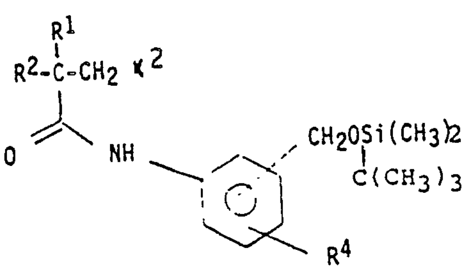

(V)

b) en formant une N-aryl-azétidinone de formule :

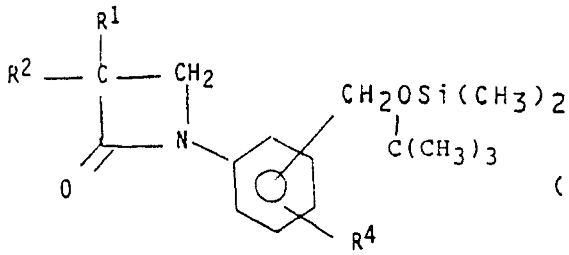

(VI)

par cyclisation de cet halopropionanilide de formule (V), et

c) en transformant cette N-aryl azétidinone de formule (VI) en N-aryl azétidinone de formule (II) par réaction avec un mélange d'acide fluorhydrique et d'eau.

15. Procédé de préparation d'une N-aryl-azétidinone répondant à la formule :

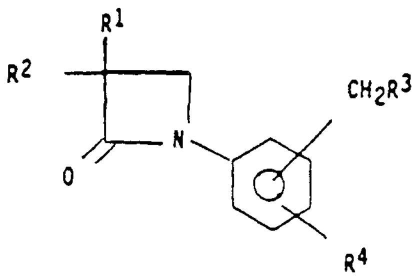

(I)

dans laquelle :

- $R^1$ et $R^2$ qui peuvent être identiques ou différents représentent un atome de F, Br, Cl ou I, ou un radical de formule $CF_3$, $COOR^5$, CN ou $CONHR^5$ avec $R^5$ représentant un radical alkyle ou aryle,
- $R^3$ représente un atome de brome, et
- $R^4$ représente un atome d'hydrogène ou un radical choisi parmi les radicaux de formule $COOR^7$, $CONHR^7$, $NO_2$, $CF_3$, CN et $SO_2R^7$, avec $R^7$ représentant un atome d'hydrogène ou un radical alkyle ou aryle et n étant un nombre entier de 1 à 18,

caractérisé en ce qu'il consiste à faire réagir une N-aryl- azétidinone de formule :

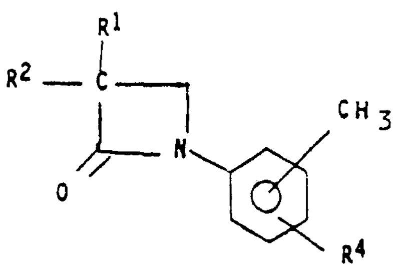

(VII)

dans laquelle $R^1$, $R^2$ et $R^4$ ont la signification donnée ci-dessus avec le N-bromosuccinimide.

16. Composition pharmaceutique comprenant un inhibiteur d'élastase, caractérisée en ce que cet inhibiteur d'élastase est une N-aryl-azétidinone de formule :

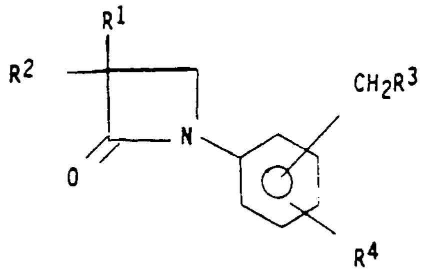

(I)

dans laquelle - $R^1$ et $R^2$ qui peuvent être identiques ou différents représentent un atome de F, Br, Cl ou I, ou un radical de formule $CF_3$, $COOR^5$, CN, $CONHR^5$ ou $COR^5$ avec $R^5$ représentant un radical alkyle ou aryle,

- $R^3$ représente un atome de chlore, de brome ou d'iode, ou un radical de formule $OC(O)R^6$, $OSO_2R^6$, $OP(O)R^6{}_2$ ou $S^+R^6{}_2$ avec $R^6$ représentant un radical alkyle, perfluoroalkyle ou aryle, et

- $R^4$ représente un atome d'hydrogène ou un radical choisi parmi les radicaux alkyle et les radicaux de formule $COOR^7$, $CONHR^7$, $NO_2$, $CF_3$, CN, $SO_2R^7$, $(CH_2)_nOR^7$ et $OR^7$ avec $R^7$ représentant un atome d'hydrogène ou un radical alkyle ou aryle et n étant un nombre entier de 1 à 18.

17. Composition pharmaceutique selon la revendication 16, caractérisée en ce que $R^4$ est un atome d'hydrogène et $R^3$ est Cl , Br ou $OSO_2CH_3$.

18. Composition pharmaceutique selon l'une quelconque des revendications 16 et 17, caractérisé en ce que $R^1$ est F et $R^2$ est F ou Br.

19. Composition pharmaceutique selon l'une quelconque des revendications 16 et 17, caractérisée en ce que $R^1$, $R^2$ et $R^3$ représentent Cl.

Office européen des brevets

# RAPPORT DE RECHERCHE EUROPEENNE

Numero de la demande

EP 89 40 3512

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | CHEMISTRY LETTERS, no. 3, Mars 1982, pages 333-336, The Chemical Society of Japan; M. WAKSELMAN et al.: "Selective cleavage of ester and ether functions with BBr3 or SiX in substituted N-arylazetidinones" * En entier * | 1-3,5,9,14 | C 07 D 205/08<br>A 61 K 31/395//<br>C 07 F 7/18 |
| D,Y | EUROPEAN JOURNAL OF MEDICINAL CHEMISTRY, vol. 18, no. 4, 1983, pages 307-314; M. ZRIHEN et al.: "N-aryl azétidinones substituées, inhibiteurs potentiels de beta-lactamases" * En entier; page 310, tableau I; page 308, formule 7b; page 313, tableau IV * | 1-3,9, 14-16 | |
| A | ZEITSCHRIFT FÜR NATURFORSCHUNG, vol. 40b, no. 1, partie B, janvier 1985, pages 115-116, Verlag der Zeitschrift für Naturforschung, Tübingen, DE; K.-H. ONGANIA: "Synthese von 3,4-Benzo-1-oxacephen" * Pages 115-116; figures 7,8,9,11-14 * | 1,9,14 | |

DOMAINES TECHNIQUES RECHERCHES (Int. Cl.5)

C 07 D 205/00
C 07 F 7/00
C 07 F 9/00
A 61 K 31/00

Le présent rapport a été établi pour toutes les revendications

| Lieu de la recherche | Date d'achèvement de la recherche | Examinateur |
|---|---|---|
| LA HAYE | 19-03-1990 | CHOULY J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)